Europäisches Patentamt

European Patent Office

Office européen des brevets

(11) Numéro de publication: **0 337 874**
**A1**

## (12) DEMANDE DE BREVET EUROPEEN

(21) Numéro de dépôt: **89400988.5**

(22) Date de dépôt: **11.04.89**

(51) Int. Cl.⁴: **A 61 B 17/10**
A 61 B 17/08

(30) Priorité: **14.04.88 FR 8804945**

(43) Date de publication de la demande:
**18.10.89 Bulletin 89/42**

(84) Etats contractants désignés:
**BE CH DE ES GB IT LI SE**

(71) Demandeur: **Laboureau, Jacques-Philippe**
**24 rue de la Fontaine Billenois**
**F-21000 Dijon (FR)**

**Comte, Georges**
**27bis Rue des Monts de Vigne**
**F-21000 Dijon (FR)**

(72) Inventeur: **Laboureau, Jacques-Philippe**
**24 rue de la Fontaine Billenois**
**F-21000 Dijon (FR)**

**Comte, Georges**
**27bis Rue des Monts de Vigne**
**F-21000 Dijon (FR)**

(74) Mandataire: **Bruder, Michel**
**Cabinet Michel Bruder Conseil en Brevets 10, rue de la**
**Pépinière**
**F-75008 Paris (FR)**

(54) **Agrafe chirurgicale de suture cutanée et outil pour sa mise en oeuvre.**

(57) La présente invention concerne une agrafe chirurgicale de suture cutanée est constituée par un morceau de fil en un matériau rigide mais déformable, notamment métallique, symétrique par rapport à un plan, terminé par deux pointes (1i) et présentes, à l'état ouvert, une forme sinueuse.

Pour limiter la pénétration des pointes (1i) et améliorer la répartition de la pression sur les différentes couches cutanées, l'agrafe comprend sucessivement, en partant du centre de l'agrafe et de chaque côté du plan de symétrie (xy) de l'agrafe, une branche rectiligne (1b, 5a), un premier coude (1c) à concavité tournée vers la peau dans laquelle doit être engagée l'agrafe, un deuxième coude intermédiaire (1e,5e) à convexité tournée vers la peau et un troisième coude (5g) à concavité tournée vers la peau, ce troisième coude (1g,5g) se prolongeant par une jambe extrême (1h,5h), terminée par la pointe (1i,5i) de l'agrafe et qui est inclinée d'un angle d'environ 45° par rapport à la direction générale d'extension de l'agrafe (1,5).

Fig.1

**Description**

La présente invention concerne une agrafe chirurgicale de suture cutanée et un outil pour sa mise en oeuvre.

On connaît déjà des agrafes chirurgicales en tôle préformée qui sont posées une à une à l'aide d'une sorte de brucelle et qui présentent les inconvénients d'être peu pointues, encombrantes et par conséquent de ne pas se prêter à une mise en chargeur pour une utilisation en agrafeuse. Par contre l'un de leurs avantages est constitué par un arrêt sur la pointe qui permet de limiter leur pénétration.

On connaît par ailleurs d'autres agrafes chirurgicales en fil métallique préformées qui sont utilisées avec une agrafeuse stérile, généralement jetable. Ces agrafes, mieux affûtées et moins encombrantes que les agrafes en tole préformée, se ferment approximativement suivant la forme d'un anneau à la manière d'un fil de suture noué traditionnellement. De ce fait elles présentent l'inconvénient de n'exercer de pression qu'au point de pénétration de leur pointe sans tenir compte de l'anisotropie constituée par la présence des berges d'une plaie. On connaît toutefois un mode de suture à fil pratiqué en chirurgie esthétique et dite de Blair-Donati qui est réalisé en disposant le noeud de fixation sur une seule berge de la plaie, exigeant donc que le fil entre et sorte par la même berge ce qui produit quatre traces de pénétration du fil autour de la plaie. Ce type de suture répartit la pression exercée de telle sorte que les couches profondes de la peau recoivent une pression égale ou supérieure à celle des couches superficielles. La tension normale de la peau n'écarte pas ces couches profondes et la cicatrisation se réalise aussi bien en profondeur qu'en superficie de telle sorte que, lors de la libération des points les couches profondes servent de charnière et que les couches superficielles, alors légèrement en compression continuent leurs cicatrisations dans de meilleures conditions.

La présente invention vise à remédier à ces inconvénients des agrafes chirurgicales connues en procurant une agrafe dont la forme est conçue de manière à limiter la pénétration de ses pointes en évitant ainsi de former un anneau et d'assurer en même temps une meilleure répartition de la pression sur les différentes couches cutanée et soucutanée entre les pointes de l agrafe et son dos.

A cet effet cette agrafe chirurgicale de suture cutanée constituée par un morceau de fil en un matériau rigide mais déformable, notamment métallique, symétrique par rapport à un plan, terminé par deux pointes et présentant à l'état ouvert, une forme sinueuse,est caractérisée en ce qu'elle comprend successivement, en partant du centre de l'agrafe et de chaque côté du plan de symétrie de l'agrafe, une branche rectiligne , un premier coude à concavité tournée vers la peau dans laquelle doit être engagée l'agrafe, un deuxième coude intermédiaire à convexité tournée vers la peau et un troisième coude à concavité tournée vers la peau, ce troisième coude se prolongeant par une jambe extrême , terminée par la pointe de l'agrafe et qui est inclinée d'un angle d'environ 45° par rapport à la direction géné rale d'extension de l'agrafe .

L'invention a également pour objet un outil pour la mise en oeuvre d'une telle agrafe chirurgicale de suture cutanée, comportant une enclume sur laquelle est appliquée la partie centrale de l'agrafe et un couteau mobile venant en contact avec les parties latérales de l'agrafe afin de les recourber autour de l'enclume, caractérisé en ce qu'il comprend, dans sa face qui est tournée vers l'enclume,un évidement de largeur supérieure à celle de l'enclume et déliminant, de part et d'autre, des faces d'appui venant en contact en premier lieu, au cours du mouvement de déformation de l'agrafe autour de l'enclume entrainant sa ferme ture, avec les troisièmes coudes externes de l'agrafe.

On décrira ci-après,à titre d'exemples non limitatifs, diverses formes d'exécution de la présente invention, en référence au dessin annexé sur lequel .

La figure 1 est une vue en élévation d'une agrafe chirurgicale suivant l'invention telle qu'elle se présente avant sa mise en place pour suturer une plaie.

Les figures 2 et 3 sont des vues similaires de l'agrafe respectivement déformée en position intermédiaire à plat et en position finale fermée.

La figure 4 est une vue en élévation d'une variante d'exécution de l'agrafe.

Les figures 5,6,7 sont des vues semblables illustrant la déformation progressive de l'agrafe de la figure 4 au cours de sa mise en place.

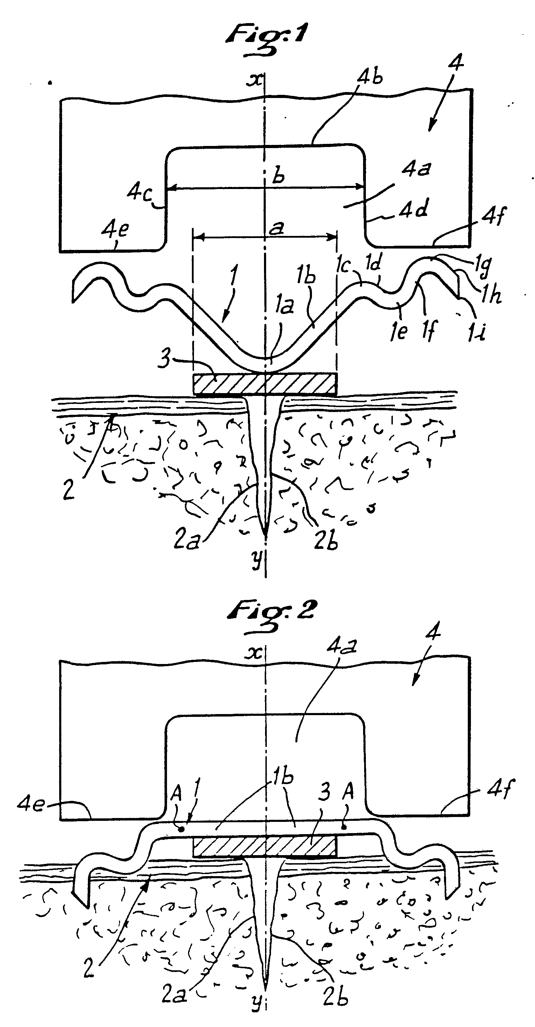

L'agrafe chirurgicale de suture cutanée suivant l'invention est représentée sur la figure 1 à 1 état ouvert, c'est-à-dire non déformée et elle présente alors une forme générale en V à angle obtus ouvert vers le haut. Cette agrafe 1 qui est symétrique par rapport à un plan vertical xy, est destinée à suturer une plaie dans la peau 2 délimitée par deux berges 2a,2b. Pour assurer cette suture l'agrafe 1 est fermée en utilisant un outil comprenant une enclume horizontale 3, de section droite verticale rectangulaire, située sous l'agrafe et en contact avec la surface de la peau, l'agrafe 1 étant en appui sur l'enclume 3 par son dos central arrondi 1a, à convexité tournée vers le bas et un couteau 4 situé au-dessus de l'agrafe 7, l'enclume 3 et le couteau 4 étant chacun symétriques par rapport au plan vertical xy.

Comme on peut le voir sur la figure 1, l'agrafe chirurgicale 1 présente de chaque côté, en partant de son dos central arrondi 1a, une branche rectiligne 1b inclinée vers le haut, un coude 1c à concavité tournée vers le bas, d'angle un peu supérieur à 90°, une jambe courte 1d inclinée vers le bas, un deuxième coude 1e à concavité tournée vers le haut, d'angle un peu inférieur à 90°, une jambe intermé diaire 1f, un troisième coude 1g à concavité tournée vers le bas, d'angle un peu inférieur à 90° et une jambe extrême 1h se terminant par une face en biseau constituant une pointe 1i. Cette jambe extrême 1h forme, avec l'horizontal, c'est-à-dire avec la direction générale d'extension de l'agrafe, un angle sensiblement égal à 45°.

L'enclume 3 qui est située sous l'agrafe 1, a une largeur a qui détermine, en combinaison avec le couteau supérieur 4, la largeur hors tout de l'agrafe chirurgicale 1 une fois celle-ci fermée. Le couteau 4 présente, dans la partie centrale de sa face inférieure, un évidement rectangulaire 4a délimité par un fond horizontal supérieur 4b et deux flancs latéraux et verticaux 4c et 4d La largeur b de l'évidement 4a, c'est-à-dire la distance entre les flancs latéraux et verticaux 4c,4d est un peu supérieure à la largeur a de l'enclume 3.

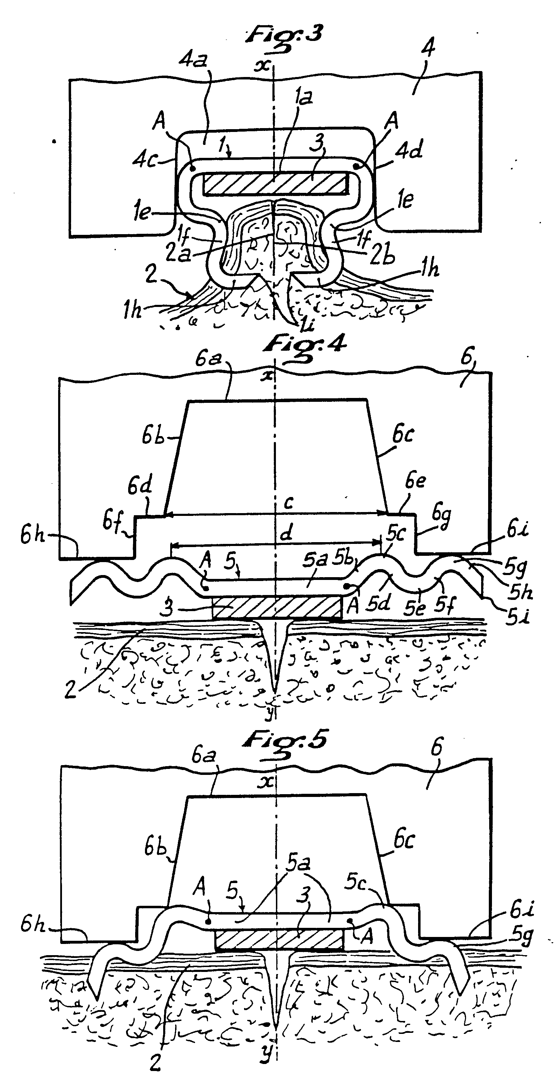

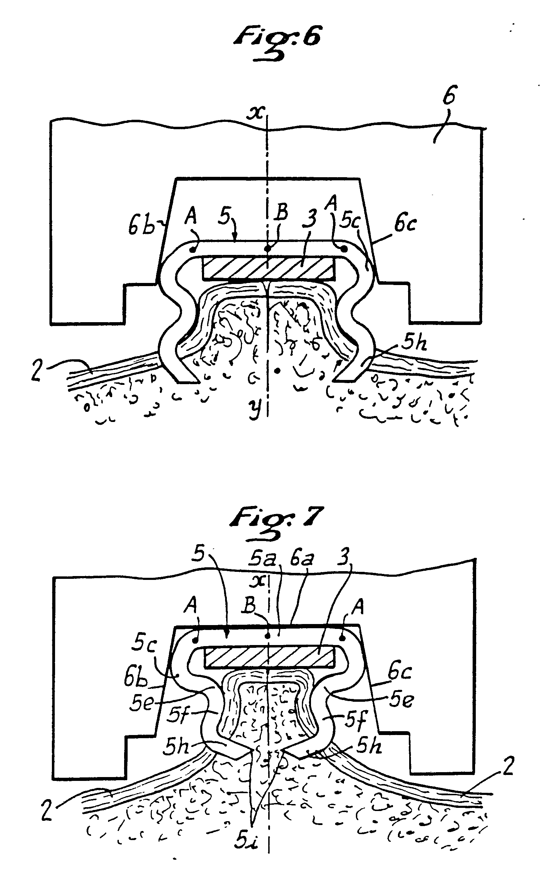

Pour la mise en place de l'agrafe 1 afin de suturer une plaie se trouvant en dessous d'elle, on provoque un mouvement de descente du couteau 4, l'enclume 3 restant fixe et maintenant le dos courbe central 1a de l'agrafe 1. Au cours du mouvement de descente du couteau 4, celui-ci vient en contact, par ses faces inférieures 4e,4f, avec les coudes 1g de l'agrafe 1 et à partir de ce moment le couteau 4 provoque une déformation de l'agrafe 1 qui est aplatie progressivement pour arriver à la position intermédiaire illustrée sur la figure 2 dans laquelle l'agrafe est horizontale. Lors de la poursuite du mouvement vers le base du couteau 4 les jambes 1b de l'agrafe 1 se trouvent alors retenues contre la face supérieure de l'enclume 3, sur la plus grande partie de leur longueur, et leurs parties extrêmes, par lesquelles elles se raccordent aux coudes 1c, sont alors déformées vers le bas en pivotant autour des points A. Ces parties extrêmes sont recourbées de 90° vers le bas lorsque l'agrafe est fermée (figure 3) et dans cette position elles s'étendent verticalement à une certaine distance des côtés verticaux de l'enclume 3. Du fait de ce mouvement de pivotement des parties extrêmes des jambes 1b autour des points A le reste de l'agrafe, constitué par ses parties 1c-1i, pivote également autour des points A en direction du plan vertical de symétrie xy, jusqu'à la position de fermeture illustrée sur la figure 3. Dans cette position de fermeture les deux jambes extrêmes 1h, terminées par les pointes 1i, s'étendent sensiblement horizontalement l'une vers l'autre en étant ancrées dans les deux berges 2a,2b de la plaie. Par ailleurs la jambe 1f et le coude 1e constituent de chaque côté, un talon limitant la pénétration de la pointe 1h,1i, d'une part, et transmettant, d'autre part, une pression mécanique répartie entre la pointe 1h,1i et le dos 1a de l'agrafe qui peut présenter une forme quelconque. Lorsque l'agrafe est fermée les deux jambes 1f convergent légèrement l'une vers l'autre, vers le haut, et elles contribuent efficacement au resserrement des berges 2a,2b de la plaie 4. Par ailleurs, d'après la description qui précède, on peut voir que chaque berge cutanée 2a,2b est tenue en deux points à savoir en un premier point profond, à l'endroit de la pointe 1h, 1i, et d'autre part en un point relativement superficiel à l'endroit du talon constitué par la jambe 1f et le coude 1e. Ceci assure l'affrontement avec son homologue controlatéral, comme dans le cas d'un point dit de Blair-Donati. Par ailleurs du fait de la forme sinueuse de l'agrafe, celle-ci ne peut pas tourner sur elle-même comme cela est le cas des agrafes qui, une fois fermées, sont voisines d'un cercle et qui présentent l'inconvénient que leur rotation possible crée des cisaillements néfastes à la cicatrisation idéale.

Dans la variante d'exécution de l'invention représentée sur la figure 4 l'agrafe chirurgicale 5 est sensiblement plane au repos en étant symétrique par rapport au plan vertical xy. De chaque côté de ce plan et en partant de celui-ci l'agrafe 5 comprend successivement une branche rectiligne horizontale 5a s'étendant au-dessus de l'enclume 3, une courte jambe 5b inclinée vers le haut, un coude 5c à concavité tournée vers le bas, d'un angle sensiblement égal à 90°, une courte jambe 5d, un coude 5e à concavité tournée vers le haut, d'un angle sensiblement égal à 90°, une courte jambe 5f inclinée vers le haut, un coude 5g à concavité tournée vers le bas, d'un angle sensiblement égal à 90°, et une jambe extrême 5h inclinée vers le bas, d'un angle d'environ 45° par rapport à l'horizontale, et terminée par une face en biseau, sensiblement verticale, délimitant une pointe 5i. Avec l'enclume 3 coopère un couteau mobile 6 présentant, dans sa face inférieure, un évidement étagé comportant une partie supérieure ou interne à section verticale trapézoïdale et une partie inférieure ou externe à section verticale rectangulaire. La partie supérieure à section verticale trapézoïdale est délimitée par un fond horizontal supérieur 6a et deux faces latérales inclinées 6b,6c qui se raccordent respectivement à des faces horizontales 6d,6e, s'étendant vers l'extérieur, constituant les faces supérieures de l'évidement rectangulaire inférieur et qui sont elles-mêmes prolongées vers le bas par des faces latérales verticales 6f,6g débouchant dans la face inférieure du couteau 6. Cette face inférieure est de ce fait limitée à deux parties latérales 6h,6i s'étendant horizontalement. La largeur c de la grande base inférieure de la partie trapézoïdale supérieure de l'évidement, c'est-à-dire la distance entre les arêtes de raccordement entre les faces latérales inclinées 6b,6c et les faces horizontales 6d,6e, est choisie égale ou légèrement supérieure à la distance d entre les sommets des deux coudes 5c de l'agrafe 5. Par ailleurs les deux faces inférieures horizontales 6h,6i du couteau 6 sont écartées l'une de l'autre d'une distance telle que lors du mouvement du couteau 6 vers l'enclume 3 elles viennent en contact avec les sommets des coudes externes 5g de l'agrafe 5 à plat sur l'enclume 3. A partir du moment où ce contact est établi, les parties latérales de l'agrafe 3 sont repliées autour des points A de raccordement entre les jambes 5a et 5b. Les jambes horizontales 5a restent plaquées sur la face supérieure de l'enclume 3 pendant qu'a lieu le pliage du reste de l'agrafe. A un certain moment de la poursuite du mouvement de descente du couteau 6, les faces horizontales intermédiaires 6d,6e viennent en contact avec les coudes internes 5c comme il est représenté sur la figure 5. A partir de ce moment la déformation de l'agrafe est assurée par les efforts exercés vers le bas par les faces horizontales intermédiaires 6d,6e sur les coudes internes 5c. Ces coudes sont ainsi progressivement repliés en direction du plan vertical xy, en enveloppant l'enclume 3, et à un certain moment, comme il est représenté sur la figure 6, ces coudes partiellement repliés 5c s'engagent dans la grande base inférieure de la partie trapézoïdale de l'évidement, en étant en contant alors avec les faces latérales inclinées 6b,6c de celui-ci. A partir de ce moment la déformation de l'agrafe est assurée par ces faces inclinées 6b,6c qui, au cours du mouvement de descente du couteau 6, repoussent progressivement les deux coudes 5c en direction de l'enclume 8. Cette déformation conduisant la fermeture de l'agrafe se poursuit jusqu'à ce que le fond supérieur 6a de l'évidement trapézoïdal vienne en contact avec les jambes horizontales 5a de l'agrafe 5 en appui sur l'enclume 3. A ce moment l'agrafe 5 est totalement fermée comme il est représenté sur la figure 7. Dans cette position de fermeture les jambes extrêmes 5h sont légèrement inclinées vers le bas, l'une vers l'autre et vers le plan vertical de symétrie xy, leurs pointes 5i étant proches l'une de l'autre, de part et d'autre du plan xy. Là encore les jambes 5f et les coudes 5e constituent des talons analogues aux talons 1f,1e de l'agrafe 1 précédemment décrite et jouant le même rôle.

Avec l'agrafe 5 qui vient d'être décrite et dont la fermeture est assurée, à la fin, par le contact de l'agrafe avec les faces internes 6b,6c de la partie supérieure trapézoïdale du couteau 6, il est nécessaire de réaliser une course importante de ce couteau 6 pour une course donnée des pointes 5i de l'agrafe. Par ailleurs ceci permet d'utiliser une agrafeuse réglable évitant de réaliser plusieurs tailles d'agrafes. On peut ainsi fermer plus ou moins l'agrafe en réglant en conséquence la course de l'agrafeuse.

On notera que lors de la pose de l'agrafe 5 ses pointes 5i pivotent autour des points A en décrivant une trajectoire engageante qui a tendance à descendre le dos de l'agrafe, constitué par les jambes 5a, sur la suture. Par contre à l'ouverture de l'agrafe le centre de rotation est le point B situé au milieu des jambes 5a, qui permet une trajectoire neutre des pointes 5i en évitant ainsi le traumatisme à l'extraction.

Les différentes formes de l'agrafe à plat et de l'agrafe fermée sont soumises à une autre contrainte qui est celle de permettre l'empilage des agrafes de telle sorte que le pas des agrafes ne dépasse pas 1,4 fois le diamètre du fil les constituant, afin de présenter des chargeurs d'agrafe autorisant le maximum de quantité sous le minimum de volume. Les agrafes 1 en forme de V ou 5 plates., telles que décrites ci-dessus, se prêtent particulièrement bien à une telle imbrication.

## Revendications

1.- Agrafe chirurgicale de suture cutanée constituée par un morceau de fil en un matériau rigide mais déformable, notamment métallique, symétrique par rapport à un plan, terminé par deux pointes et présentant, à l'état ouvert, une forme sinueuse, caractérisée en ce qu'elle comprend sucessivement, en partant du centre de l'agrafe et de chaque côté du plan de symétrie (xy) de l'agrafe, une branche rectiligne (1b 5a), un premier coude (1c) à concavité tournée vers la peau dans laquelle doit être engagée l'agrafe, un deuxième coude intermédiaire (1e,5e) à convexité tournée vers la peau et un troisième coude (5g) à concavité tournée vers la peau, ce troisième coude (1g,5g) se prolongeant par une jambe extrême (1h,5h), terminée par la pointe (1i,5i) de l'agrafe et qui est inclinée d'un angle d'environ 45° par rapport à la direction générale d'extension de l'agrafe (1,5).

2.- Agrafe chirurgicale suivant la revendication (1) caractérisée en ce que l'agrafe (1) a une forme générale en V d'angle obtus et elle comporte un dos central arrondi (1a), à l'endroit du sommet du V, qui se raccorde aux deux branches rectilignes (1b).

3.- Agrafe chirurgicale suivant la revendication (1) caractérisée en ce que l'agrafe (5) a une forme plane à l'état ouvert et ses deux branches centrales rectilignes (5a) sont coaxiales.

4.- Outil pour la mise en oeuvre d'une agrafe chirurgicale de suture cutanée suivant l'une quelconque des revendications (1) à (3) comportant une enclume sur laquelle est appliquée la partie centrale de l'agrafe et un couteau mobile venant en contact avec les parties latérales de l'agrafe afin de les recourber autour de l'enclume, caractérisé en ce qu'il comprend, dans sa face (4e,4f;6h,6i), qui est tournée vers l'enclume (3) un évidement de largeur supérieure à celle de l'enclume et délimitant, de part et d'autre, des faces d'appuis (4e,4f;6h,6i) venant en contact en pre mier lieu, au cours du mouvement de déformation de l'agrafe (1,5) autour de l'enclume (3) entraînant sa fermeture, avec les troisième coudes externes (1g,5g) de l'agrafe.

5.- Outil suivant la revendication (4) caractérisé en ce que l'évidement (4a) du couteau (4) a une forme rectangulaire, il est délimité par un fond (4b) et deux flancs latéraux (4c,4d), la distance entre ces flancs latéraux (4c,4d) étant supérieure à la largeur (a) de l'enclume (3).

6.- Outil suivant la revendication (4) caractérisé en ce que l'évidement du couteau (6) comprend une partie supérieure ou interne à section verticale trapézoidale qui est délimitée par un fond horizontal supérieur (6a) et deux faces latérales inclinées (6b,6c), et une partie inférieure ou externe à section verticale rectangulaire qui comprend deux faces horizontales (6d,6e), s'étendant vers l'exté rieur, constituant les faces supérieures de l'évidement rectangulaire inférieur et qui sont elle-mêmes prolongées vers le bas par des faces latérales verticales (6f, 6g) débouchant dans la face inférieure du couteau (6) laquelle est de ce fait limitée à deux parties latérale (6a,6i) s'étendant horizontalement.

7.- Outil suivant la revendication (6) caractérisé en ce que les deux faces inférieures horizontales (6h,6i) du couteau (6) sont écartées l'une de l'autre d'une distance telle que lors du mouvement du couteau (6) vers l'enclume (3) elles viennent en contact avec les sommets des troisièmes coudes externes (5g) de l'agrafe (5) à plat sur l'enclume (3).

8.- Outil suivant l'une quelconque des revendications (6) et (7) caractérisé en ce que la largeur de la grande base inférieure de la partie trapézoïdale supérieure de l'évidement, c'est-à-dire la distance entre les arêtes de raccordement entre les faces latérales inclinées (6b,6c) et les faces horizontales (6d,6e), est égale ou légèrement supérieure à la distance $d$ entre les sommets des deux premiers coudes (5c) de l'agrafe (5).

9.- Outil suivant l'une quelconque des revendications (6) à (8) caractérisé en ce qu'il permet, par la largeur course du couteau (6) et la forme trapézoïdale de sa partie supérieure (6a,6b,6c) un réglage de la fermeture de l'agrafe selon la nature de la peau à suturer, évitant l'usage d'agrafes de tailles différentes.

## Fig. 1

## Fig. 2

Fig:3

Fig:4

Fig:5

Fig. 6

Fig. 7

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 094 752 (J. BLAKE et al.)<br>* Page 20, ligne 14 - page 21, ligne 10; figures 31,32 *<br>--- | 1,4 | A 61 B 17/10<br>A 61 B 17/08 |
| A | EP-A-0 253 629 (MINNESOTA MINING AND MANUFACTURING CO.)<br>* Résumé; figures 5,6 *<br>--- | 1,4 | |
| A | EP-A-0 180 820 (AMERICAN CYANAMID CO.)<br>* Figure 9; page 5, lignes 28-33 *<br>----- | 1 | |
| | | | **DOMAINES TECHNIQUES RECHERCHES (Int. Cl.4)**<br><br>A 61 B |

Le présent rapport a été établi pour toutes les revendications

| Lieu de la recherche | Date d'achèvement de la recherche | Examinateur |
|---|---|---|
| LA HAYE | 18-05-1989 | NEILL M.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...................................................................
& : membre de la même famille, document correspondant

EPO FORM 1503 03.82 (P0402)